# NEW EUROPEAN PATENT SPECIFICATION

(11) **EP 0 460 020 B2**
(45) Date of publication and mention of the opposition decision: **17.09.1997**
(45) Mention of the grant of the patent: 20.04.1994
(21) Application number: 90903612.1
(22) Date of filing: 23.02.1990
(51) Int. Cl.: A61K 9/06, A61K 47/36

(54) **DRUG DELIVERY COMPOSITIONS**
ARZNEISTOFFABGABE-ZUSAMMENSETZUNGEN
COMPOSITIONS POUR L'ADMINISTRATION DE MEDICAMENTS

(30) Priority: 25.02.1989 GB 8904370
(43) Date of publication of application: 11.12.1991
(73) Proprietor: DANBIOSYST UK LIMITED, University Boulevard Nottingham NG7 2QJ (GB)
(72) Inventor: ILLUM, Lisbeth, Nottingham NG7 1BA (GB)
(74) Representative: Bassett, Richard Simon
(86) International application number: GB9000291
(87) International publication number: WO9009780

(56) References cited:
- DE-A- 2 414 599
- DE-A- 3 200 766
- Biological Abstracts, volume 30, no. 11, Y. Sawayanagi et al.: "Directly compressed tablets containing chitin or chitosan in addition to mannitol", pages 4216-4218
- Chemical Abstracts, volume 112, 1990 (Colombus, Ohio, US), A. Manosroi et al.: "The entrapment of a human insulin-DEAE dextran complex in different compound liposomes" see abstract 84024c & Drug Dev. Ind. Pharm. 1989, 15(14-16), 2531-46
- Chem. Abstracts, Vol. 103, 1985, page 316, C.A. 103: 92752t
- M. Longer et al., Journ. of Pharmaceutical Sciences, 74, 406 (1985)
- J.R. Robinson et al., Ann. N.Y. Acad. Sci., New York, 507, 307-314 (1987)
- G. Losse et al., Wiss. Z. Univ. Dresden, 37(3), 141-144
- L.A. Potempa et al., Molec. Immunology, 20, 501-509 (1983)
- Drug Development and Industrial Pharmacy, Ed. C.T. Rhodes, Vol. 10, pages 613-622 (1984)
- Pharm. Res., Vol. 11, No. 8, pages 1186-1189, 1994, Illum et al.
- Pharm. Res., Vol. 11, No. 9, pages 1358-1361, 1994, Artursson et al.
- Prog. Colloid Polym. Sci., 94, 66-73, pages 66-73, Fiebrig et al.
- Posters/Europ. J. Pharm. Sci. 2 (1994), 117-194, PAGE +185, abstract P265 + Figure, I. Fiebrig et al.
- J. Physiol. (1992), Vol. 446, page 31, G.T.A. McEwan et al.
- Biochim. Biophys. Acta., May 14 1993, 1148, pages 51-60. G.T.A. McEwan et al.

## Description

The present invention relates to drug delivery compositions and more particularly to compositions which provide for the uptake of active drug material across mucosal surfaces, such as the vagina, colon or the nasal cavity.

A major problem in drug delivery is the effective absorption of high molecular weight material such as proteins and peptides across biological membranes. Normally such molecules are not taken up by the body if administered to the gastrointestinal tract, the buccal mucosa, the rectal mucosa, the vaginal mucosa or the intranasal mucosa. Recent studies with insulin have demonstrated that the absorption of such a compound can be increased if it is given together with a so-called absorption enhancer. These absorption enhancing materials have included surfactants of the non-ionic type as well as various bile salt derivatives. An increased permeability of membranes in the presence of these types of surfactant materials is obtained and the literature in the field of gastroenterology contains a wide range of such absorption promoters. (For a review see Davis *et al* (editors), Delivery Systems for Peptide Drugs, Plenum Press, New York, 1987.) However, such materials will probably not be acceptable for the chronic administration of pharmacological agents because of their irritant effects on membranes. This includes not only the non-ionic variety of surface active agents but also bile salts and bile salt derivatives (e.g. fusidic acid).

EP-A-023 359 and EP-A-122 023 describe a powdery pharmaceutical composition for application to the nasal mucosa and methods for administration thereof. The pharmaceutical composition allows polypeptides and derivatives thereof to be effectively absorbed through the nasal mucosa. Similarly, US-A-4 226 848 describes a method for administering a powdery medicament to the nasal mucosa where the preferred composition has mucoadhesive properties.

EP-A-230 264 describes an aqueous nasal drug delivery system for vaccines containing a high molecular weight drug, a gelling agent (e.g. hydroxyethylcellulose) and in some cases other additives (e.g. surfactants, glycerol and polyethyleneglycol) but, again, the composition is administered as a powder.

Microsphere-containing formulations have been described in WO 88/09163. The formulations contain certain enhancers to aid effective penetration of the mucosa by the drug. Our co-pending application WO 89/03207 further describes formulations which do not require an enhancer. These formulations may comprise drug-containing microcapsules which are coated with DEAE-dextran.

DEAE-dextran has been proposed for use in oral drug delivery formulations, where it is believed to interact with gastrointestinal mucins (Anderson, M.T. *et al*, oral presentation at a meeting of the Society for Experimental Biology, 24-29 July 1988, Manchester, U.K.) and has been delivered to the nasal cavities of rabbits as a model compound to study the absorption of peptides of differing sizes (Maitani, Y., *et al*. Int. J. Pharm. 1989, 49, 23-27).

Igawa *et al* (1988 Chem. Pharm. Bull. 36(8) 3055-3059) administered human interferon-β intranasally to rabbits with a DEAE-dextran excipient. The dextran part of the latter had an average molecular weight of 9000 and did not enhance the absorption of the drug, and the authors concluded that low MW excipients were to be preferred to high MW components. In view of this, it is surprising to find, as we now have, that a solution or dispersion of relatively high MW DEAE-dextran or other polycationic substances such as chitosan can form the basis of an improved formulation which does not require other enhancers, although the presence of other enhancers may further improve the performance of the compositions.

GB-A-2 092 002 (equivalent to DE-A-3200766) discloses magnesium- and calcium-cheating compounds for enhancing the absorption of drugs through a digestive organ. Such compounds included polyamino acids. Sawayanagi *et al* (1982) Chem. Pharm. Bull. 30(11), 4216-4218 and Biological Abstract 75074688 disclosed the use of chitosan to bind the ingredients of tablets for retention in the mouth. Delivery to non-oral mucosal surfaces was not disclosed.

EP-A-0368253, available as prior art by virtue of Article 54(3) EPC, discloses a film-forming delivery system for delivery of an active compound to a desired topical site. The delivery system comprises at least one aminopolysaccharide derivative, either a chitosonium polymer or a chitosan derivative, and upon delivery forms a substantive gas permeable film from which the active compound is available at the designated site.

One aspect of the invention provides the use of a polymeric substance having a plurality of cationic groups and having a molecular weight of 10000 or more (hereinafter "a polycationic substance") in the manufacture of a composition for administration to mucosa, the composition further comprising a pharmacologically active compound, wherein the polycationic substance provides for uptake of the active compound across the mucosal surface and systemic distribution of the active compound and wherein (i) the substance is not a polyamino acid which chelates calcium or magnesium ions, (ii) the composition does not consist of microcapsules coated with DEAE-dextran, (iii) if for administration to gut mucosa, the composition does not consist of the active compound and a solution of DEAE-dextran and (iv), if in the form of a tablet for retention in the mouth, the composition does not comprise chitosan.

The polycationic substance may be present as a solution in an aqueous medium, as a dispersion in an aqueous system, as a powder or as microspheres. Preferably, such microspheres are formed from the polycationic substance itself (usually with the pharmacologically active substance incorporated as well) with or without other suitable microsphere-forming substances such as (human) serum albumin and derivatives and analogues thereof.

Preferably, the concentration of the polycationic substance in such a solution is 0.01 to 50% w/v, more preferably 0.1 to 50%, more preferably 0.2% to 30% and most preferably 0.5-15%.

Diethylaminoethyl-dextran (DEAE-dextran) is a polycationic derivative of dextran containing diethylaminoethyl groups coupled to the glucose residues by ether linkages. The parent dextran can have an average molecular weight of about 5,000 to 40 x 10⁶, but is typically about 500,000. In the context of the present invention, the term is limited to dextran of MW 10000 or more. The nitrogen content is usually approximately 3.2% which corresponds to one charged group to three glucose units. "Tandem" groups, which are introduced as the result of side reactions, result in the presence of three different basic groups in approximately equal ratios.

Chitosan is deacetylated chitin, or poly-N-acetyl-D-glucosamine. It is available from Protan Laboratories Inc, Redmond, Washington 98052, USA and, depending on the grade selected, can be soluble in water up to pH 6.0. A 1% solution of non-water soluble chitosan (Sea Cure) may be made by making a slurry (eg 2g/100 ml) in water and adding an equal volume of organic acid (eg 100 ml of 2% acetic acid) and stirring vigorously for one hour. Water-soluble chitosan (Sea Cure⁺) may dissolve without organic or inorganic acids being present.

Chitosan has previously been used to precipitate proteinaceous material, to make surgical sutures and as an immunostimulant. It has also been employed previously in oral drug formulations in order to improve the dissolution of poorly soluble drugs (Sawayanagi *et al*, Chem. Pharm. Bull., 31, 2062-2068 (1983)) or for the sustained release of drugs (Nagai *et al*, Proc. Jt. US-Jpn. Semin. Adv. Chitin, Chitosan, Relat. Enzymes, 21-39. Zikakis J.P. (ed), Academic Press. Orlando (1984)) by a process of slow erosion from a hydrated compressed matrix.

DEAE-dextran and chitosan are preferred, but further polycationic substances which may be used in the compositions of the invention include other polycationic carbohydrates such as but not limited to inorganic or organic salts of chitosan and modified forms of chitosan (especially more positively charged ones), polyaminoacids such as polylysine, polyquaternary compounds, protamine, polyimine, DEAE-imine, polyvinylpyridine, polythiodiethylaminomethylethylene (P(TDAE)), polyhistidine, DEAE-methacrylate, DEAE-acrylamide, poly-p-aminostyrene, polyoxethane, co-polymethacrylates (e.g. copolymers of HPMA, N-(2-hydroxypropyl)methacrylamide), GAFQUAT (US Pat No 3,910,862) and polyamidoamines. The polycationic substances used in the invention have a molecular weight of 10 000 or more, preferably at least 100 000 or 200 000 and most preferably about 500 000. The chitosan (or a salt thereof) preferably has an intrinsic viscosity of at least 400 ml/g, more preferably at least 500, 750 or 1000 ml/g.

If desired, other enhancers may be included in the compositions of the invention, for example lysophosphatidylcholine and generally all those mentioned in W0 88/09163. Gelling agents or viscosity-increasing substances may be added in order to help retain the formulation on the mucosa. The chitosan, in particular, may be formulated as microspheres with or without albumin.

The compositions may be prepared at a neutral pH, i.e. pH 6.5-7.5, preferably about 7.3, for example using a standard phosphate buffer or at lower pH, for example pH4, by addition of HCl to the above or by use of an alternative buffer system. However, it has been found that DEAE-dextran or chitosan in combination with at least some drugs, for example insulin and most if not all other proteins, form a complex. At lower or higher pH's, i.e. away from the isoelectric point of the polycation and the drug, this complex may be present as a true solution instead of a dispersion. This may be advantageous, although it is also the case that very low pH's are more likely to irritate or even harm the mucosa. Thus, the man skilled in the art will be able to determine the optimal pH, which may lie between 1.0 and 11.0, preferably 4.0 to 7.5, for example 4.0 to 6.0, or 9.0 to 11.0.

The said complex may be isolated. The complex (when the polycationic substance is chitosan) and its therapeutic utilities form further aspects of the invention.

The term "pharmacologically active compound" includes drugs, vaccines and components thereof (for example isolated antigens or parts thereof) and monoclonal antibodies.

The compositions may be used with drugs selected from the following non-exclusive list: insulin, calcitonins (for example porcine, human, salmon, chicken or eel) and synthetic modifications thereof, enkephalins, LHRH and analogues (Nafarelin, Buserelin, Zolidex), GHRH (growth hormone releasing hormone), nifedipin, THF(thymic humoral factor), CGRP (calcitonin gene related peptide), atrial natriuretic peptide, antibiotics, metoclopramide, ergotamine, Pizotizin, nasal vaccines (particularly AIDS vaccines, measles, rhinovirus Type 13 and respiratory syncitial virus), pentamidine and CCK (cholecystykinin).

Further drugs include: antibiotics and antimicrobial agents such as tetracycline hydrochloride, leucomycin, penicillin, penicillin derivatives, erythromycin, sulphathiazole and nitrofurazone; local anaesthetics such as benzocaine; vasoconstrictors such as phenylephrine hydrochloride, tetrahydrozoline hydrochloride, naphazoline nitrate, oxymetazoline hydrochloride and tramazoline hydrochloride; cardiotonics such as digitalis and digoxin; vasodilators such as nitro-glycerine and papaverine hydrochloride; antiseptics such as chlorhexidine hydrochloride, hexylresorcinol, dequaliniumchloride and ethacridine; enzymes such as lysozyme chloride, dextranase; bone metabolism controlling agents such as vitamin D, and active vitamin D₃; sex hormones; hypotensives; sedatives; anti-tumor agents; steroidal anti-inflammatory agents such as hydro-cortisone, prednisone, fluticasone, prednisolone, triamcinolone, triamcinolone acetonide, dexamethasone, betamethasone, beclomethasone, and beclomethasone dipropionate; non-steroidal anti-inflammatory agents such as acetaminophen, aspirin, aminopyrine, phenylbutazone, mefanamic acid, ibuprofen, diclofenac sodium, indomethacin, colchicine, and probenocid; enzymatic anti-inflammatory agents such as chymotrypsin and bromelain seratiopeptidase; anti-histaminic agents such as diphenhydramine hydrochloride, chloropheniramine maleate and clemastine; and anti-allergic agents and antitussive-expectorant antiasthmatic agents such as sodium chromoglycate, codeine phosphate, and isoproterenol hydrochloride.

The compositions can be administered via the nasal route using a nasal spray device, pressurized aerosol cannister or simple instillation means. The compositions may gel on the mucosa, at least to some extent, and this may facilitate retention of the composition on the mucosa. Formulations suitable for delivery of drugs to the colon can be subdivided into a number of technical categories known to those skilled in the art of pharmaceutical formulation. These can utilise coated solid dosage forms, such as tablets, pellets, mini-tablets, hard gelatin capsules etc or coated semi-solid preparations, such as soft gelatin capsules and the like. Enteric coated systems, based for example on methacrylate copolymers such as Eudragit L (Poly (methacrylic acid, methyl methacrylate)), are only soluble at pH 6 and above, so that the polymer only begins to dissolve on entry into the small intestine. The site of the disintegration is then dependent on the rate of intestinal transit and the amount of polymer present, a relatively thick polymer coating having been defined for delivery to the proximal colon (Hardy *et al*, Aliment. Pharmacol. Therap., 1, 273-280, (1987)). Polymers capable of providing site-specific colonic delivery can be utilised. These typically rely on the bacterial flora of the large bowel to provide enzymatic degradation of the polymer coat and hence release of the drug. A number of candidate materials appear promising, such as the azopolymers (Saffran *et al*, US Patent 4,663,308), glycosides (Friend *et al*, J. Med. Chem., 27, 261-266, (1984)) and a variety of naturally available and modified polysaccharides (Archer & Ring PCT Application GB89/00581).

Novel pulsed release technology (Magruder *et al*, US Patent 4,777,049) and the like, which permits drug delivery at a predetermined time, is now available. Such systems can be used to deliver both drug and polycationic substance, together with other additives that may alter the local microenvironment to promote drug stability and uptake, directly to the colon and do not rely on external conditions to provide *in vivo* release, except for the presence of water.

A further aspect of the invention provides a method of treating a human or other mammal by administering a composition as described above to a mucosal surface of that human or other mammal, for example the vagina, eye, colon or nasal cavity.

Embodiments of the present invention will now be described by way of example.

### EXAMPLE 1 : INSULIN PLUS DEAE-DEXTRAN

A rat *in vivo* experimental model, modified from that originally described by Hirai *et al* (1981 Int. J. Pharm., 7 317-325) and Fisher *et al* (1987 J. Pharm. Pharmacol., 39 357-362), was used to study the intranasal absorption of insulin aqueous solutions. Male Wistar rats (Bantin and Kingman) of approximate weight 200-250g, fasted overnight for about 20 hours, are anaesthetised by i.p. injection of 80 mg/kg pentobarbitone sodium (60 mg/ml Sagatal (Regd. T.M.) May and Baker) with further i.p. injections of 0.05 ml when necessary to maintain a suitable level of anaesthesia. The rats are tracheotomized, the oesophagus sealed and the carotid artery and jugular vein cannulated.

Insulin (semisynthetic human Na-insulin) solutions were prepared in 1/75 M phosphate buffer of pH 7.3 to give a concentration of 167 IU/ml and the DEAE-dextran added to give concentrations of 10% w/v, 5% w/v or 1% w/v. The DEAE-dextran used in these experiments has a molecular weight of 500 000.

It is also possible to make up a solution of 334 IU/ml of insulin in phosphate buffer and add equal volumes of the DEAE-dextran in phosphate buffer of 20, 10 or 2% strength. This will give the same end solutions. When mixing the insulin solution with the DEAE-dextran the solution becomes turbid indicating that an interaction between the insulin and the DEAE-dextran has taken place.

An insulin solution containing the Laureth-9 enhancer system was prepared in a similar way.

The insulin solution alone or the insulin solutions containing the Laureth-9 or the various concentrations of DEAE-dextran were administered nasally to rats (n = 4) at 16.7 IU/kg bodyweight using a Hamilton microsyringe. A volume of 20 µl was administered.

Blood samples of 0.2 ml were collected in Fluoride oxalate tubes from the carotid artery at 10 and 5 min. prior to the insulin administration and at 5, 15, 30, 45, 60, 90, 120, 180, 240 and 300 min. post-administration. The samples were kept for a short time on crushed ice until analysed on a Yellow Springs 23 AM glucose analyser by the glucose oxidase method.

Table 1 shows the approximate glucose levels (mmol/l) of rats given a dose of insulin in phosphate buffer and doses of insulin in phosphate buffer (pH 7.3) containing 1%, 5% or 10% DEAE-dextran measured at 120 minutes after administration. The level at the time of administration was about 3.5-4.0 mmol/l. The results show that insulin given intranasally as a simple phosphate buffer solution (pH 7.3) does not significantly lower the blood glucose level whereas the addition of the DEAE-dextran causes fast and significant decreases in blood glucose levels. The effect increases with increasing concentration of DEAE-dextran. The rats given the 10% concentration died early of hypoglycaemia. Administration of phosphate buffer alone shows a similar trend to that of the insulin solution alone, i.e. an increase in plasma glucose from about 3.5-4.0 mmol/l to about 5 mmol/l.

**Table 1**

| | Blood glucose level (mmol/l) |
|---|---|
| Insulin plus DEAE-dextran 1% | 1.6 |
| Insulin plus DEAE-dextran 5% | 1.2 |
| Insulin plus DEAE-dextran 10% | 1.0 |
| Insulin alone | 5.1 |

For comparison, the glucose levels of rats given a dose of insulin in phosphate buffer and rats given a dose of insulin in phosphate buffer containing 0.5% Laureth-9 show that this well known effective enhancer system gives a decrease in blood glucose concentration similar to the 1% DEAE-dextran (about 1.9 mmol/l at 120 mins).

### Example 2 : EFFECT OF pH ON INSULIN/DEAE-DEXTRAN SOLUTIONS

Solutions containing DEAE-Dextran 1% w/v and Na-Insulin 167 IU/ml were prepared, separately and combined, in phosphate buffer (pH 7.3) and their pH measured using a Gallenkamp pH Stick. The appearance of each solution was noted. The effect of addition of 1M sodium hydroxide solution (NaOH) or 0.1M hydrochloric acid (HCl) was determined. The two separate solutions were each clear (DEAE-D pH 6.58; Insulin pH 7.38) whereas the mixture (pH 6.65) was turbid.

The addition of 0.1M HCl to solutions of DEAE-dextran alone had no effect on solution appearance which remained clear. Solutions of Na-insulin however, became turbid when the pH reached 6.65 but cleared after further addition of acid lowered the pH to 4.14. Solutions of DEAE-dextran combined with Na-Insulin became less turbid after the addition of acid and were clear at pH 4.14. The addition of 1.0M NaOH to solutions of DEAE-dextran and Na-insulin alone had no effect on solution appearance which remained clear. Combined solutions of DEAE-dextran and Na-insulin however became less turbid as the pH increased and formed a clear solution when the pH reached 9.32. Solutions of DEAE-dextran and Na-insulin at about pH 4.0 were found to be at least as effective as those at about pH 6.6 in the rat model described above.

### EXAMPLE 3 : TOXICITY OF A COMPOSITION OF THE INVENTION

### Insulin 100IU/ml with DEAE-Dextran 5%w/v

The effects of the DEAE-dextran formulation on the nasal mucosa in rats (after 60 min incubation) were less dramatic than those of prior art surfactant enhancers. A few cells lost from the septum and turbinates were visible and mucus discharge on the dosed side resulted in a slight decrease in epithelium height. The clear cell structure was not so well defined and cytoplasmic space appeared reduced. The epithelium still appeared to be more than one cell thick (i.e. pseudostratified) and formed a continuous layer, though the arrangement of nuclei above the basement membrane was altered. Cilia were not always distinct amongst the discharged mucus.

Considerable amounts of AB staining mucus were still apparent in cells on the dosed side though there was generally not the confluent spread of filled goblet cells as on the undosed side. Some mucus was again present in the undosed cavity of some animals.

Effects of this formulation were generally restricted to the ventral half of the cavity and lateral nasoturbinate i.e. the dorsal meatus was unaffected.

### EXAMPLE 4 : (COMPARATIVE EXAMPLE) TOXICITY OF PRIOR ART COMPOSITION

### Insulin 100IU/ml with STDHF 1%w/v

As compared to DEAE-dextran 5% w/v, STDHF (sodiumtaurodihydroxyfusidate) administered in the same way to rats and incubated for 60 mins showed obvious disruption to the nasal epithelium. Large volumes of mucus were apparent together with cell loss, epithelium rearrangement and considerable reduction of epithelium height to about half that on the undosed side. Generally the full length of the dosed septum and turbinates were affected. AB staining showed that some mucus remained in many of the epithelial cells but others had discharged their whole mucus content, particularly where the epithelium was reduced to a thin single cell layer such as in the middle meatus.

Some mucus was apparent on the undosed septum or drained into the dorsal meatus, but with no cell loss. The undosed turbinates were unaffected. Epithelial height on the dosed side was consistently less than that on the undosed 'control' side.

### EXAMPLE 5 : INSULIN PLUS CHITOSAN IN THE RAT

This Example was performed to evaluate the effect of chitosan, low or medium viscosity water soluble formulations (Sea cure⁺), at different concentrations and at pH values of 4 and 7.3-7.4 on the intranasal absorption of insulin in rats (n=4).

Semisynthetic Na-insulin and chitosan (Sea cure⁺) (water soluble powder) low viscosity (l.v.)and medium viscosity (m.v) from Protan Laboratories Inc. were used.

All insulin solutions were initially made in 14.65 mM phosphate buffer of pH 7.3-7.4 prepared from 1.904 g/l Na₂HPO_{4.}2H₂O and 0.616 g/l NaH₂PO_{4.}2H₂O in double distilled water. Adjustment of the pH to 4 where necessary was made by the addition of 150 µl of 0.1M HCl per ml of solution. Each 1 mg of insulin was considered equivalent to 28IU. Double-strength insulin stock solutions were prepared freshly as follows: 159.9 IU/ml (6.74 mg/ml) for administration at pH 7.3-7.4 and 183.8 IU/ml (7.75 mg/ml) for administration at pH 4, accounting for the dilution by the addition of 0.1M HCl. The expected water content of the insulin is 15.3%.

Double strength chitosan solutions were prepared as follows: 0.2% w/v l.v. (2 mg/ml) for use at pH 7.3-7.4; 1.0% w/v l.v. (10 mg/ml) for use at pH 7.3-7.4; 0.2% w/v l.v. (2.3 mg/ml) for use at pH 4; 1.0% w/v l.v. (11.5 mg/ml) for use at pH 4; and 0.2% w/v m.v. (2.3 mg/ml) for use at pH 4.

Insulin/chitosan formulations were prepared by mixing equi-volumes of the appropriate stock insulin and chitosan solutions and the addition of 150µl/ml of 0.1M HCl where necessary. Solutions were administered intranasally to rats at 100µl/kg, corresponding to doses of 8 IU/kg insulin with 0.1 or 0.5 mg/kg l.v. chitosan or 0.1 mg/kg m.v. chitosan. A dose of 100 µl/kg of Insulin (167 IU/ml) is instilled into the nasal cavity via a microsyringe (Hamilton) and 0.61mm o.d. polypropylene tubing (Portex).

Blood samples of 150 µl (8-12 drops) were collected from the carotid artery in fluoride oxalate blood tubes at 10, 6 and 2 minutes pre-administration and 5, 10, 15, 20, 40, 60, 90, 120, 180 and 240 minutes post-administration. Fluid replacement was given in the form of 0.9% saline via the jugular vein. The glucose levels of the samples were assayed within 2 hours of being taken using the glucose oxidase method on a Yellow Springs 23AM glucose analyser.

The pH 4 solutions were not buffered systems. A suitable buffered system may be devised if desirable.

All of the formulations gave a rapid fall in blood glucose levels, the 0.5% l.v. pH 4.0 solution reducing the level from 100% to about 16% after 60 minutes. Generally, 0.5% material was more effective than 0.1% and pH 4.0 was better than pH 7.3-7.4.

### EXAMPLE 6 : INSULIN PLUS CHITOSAN IN THE SHEEP

Semi-synthetic human Na-insulin supplied by Nordisk, Gentofte was used. The water content of the sample was determined by spectrophotometry to be approximately 15%. Chitosan SEA CURE +, which is water soluble, of low (intrinsic viscosity 388 ml/g) and medium viscosity (intrinsic viscosity 1010 ml/g) were obtained from Protan Laboratories Inc. These will be referred to as CSN LV and CSN MV, respectively. Sixteen cross-bred sheep of known weight were used. The animals were not fasted prior to insulin administration. An in-dwelling Viggo secalon cannula of 1.2 mm i.d., fitted with a secalon universal flow-switch, was placed approx. 15 cm into one of the external jugular veins of each animal on the first day of the study and, whenever necessary, was kept patent by flushing it with heparinised normal saline (25 IU/ml). This cannula was removed upon the completion of the study.

An insulin solution of 19.32 mg/ml (460 IU/ml) was prepared in 14.65 mM phosphate buffer (0.476g Na₂HPO₄.2H₂O + 0.154g Na₂PO₄. 2H₂O in 250 ml water) of pH 7.3-7.4, and filtered on a 0.2 µm membrane filter (Corning 21052-25). Chitosan solutions were prepared in 14.65 mM phosphate buffer as follows: 2.3 mg/ml CSN LV, 11.5 mg/ml CSN LV, 2.3mg/ml CSN MV or 11.5 mg/ml CSN MV. Insulin/chitosan formulations were produced by mixing equal volumes of the insulin stock solution and the appropriate chitosan solution, followed by the addition of 0.15 ml of 0.166 M hydrochloric acid for each 1.0 ml of the mixture. The addition of hydrochloric acid proved necessary to ensure that the chitosan remained in solution.

The final formulations thus produced had the following composition:
- Formulation 1:: 200 IU/ml insulin + 0.1% CSN LV, pH 3.6
- Formulation 2:: 200 IU/ml insulin + 0.5% CSN LV, pH 4.4
- Formulation 3:: 200 IU/ml insulin + 0.1% CSN MV, pH 3.6
- Formulation 4:: 200 IU/ml insulin + 0.5% CSN MV, pH 4.4
The sheep were divided into 4 groups, each of 3 animals, with each sheep receiving 2.0 IU/kg insulin intranasally in the form of an aqueous solution of Formulation 1, 2, 3 or 4, corresponding to Groups 1 to 4.

For the intranasal studies, the sheep were sedated by use of an i.v. dose of ketamine hydrochloride at 2.25 mg/kg. This was intended as a counter-measure against the animal sneezing during administration. The anaesthesia lasted for about 3 minutes. Blood samples of 6 ml were collected onto crushed ice from the cannulated jugular vein of the sheep at 15 and 5 min prior to the insulin administration and at various times post-administration. Each blood sample was divided into two parts. For insulin analysis, the blood collected (4.0 ml) was mixed gently in 5 ml heparinised (Li Heparin) tubes. For glucose analysis, the blood collected (2.0 ml) was mixed gently in 5 ml fluoride oxalate tubes. The plasma was separated by centrifugation at 4°C and 3000 rpm, and then stored at -20°C awaiting insulin and glucose analysis.

The following results were obtained:

**Table 2**

| Mean blood glucose level (mmol/l) | | |
|---|---|---|
| | 5 mins post-administration | 75 mins post-administration |
| Group 1 | 3.4 | 3.0 |
| Group 2 | 3.4 | 2.5 |
| Group 3 | 3.4 | 2.6 |
| Group 4 | 3.8 | 1.8 |

## Claims

1. The use of a polymeric substance having a plurality of cationic groups having a molecular weight of 10000 or more (hereinafter "a polycationic substance") in the manufacture of a composition for administration to mucosa, the composition further comprising a pharmacologically active compound, wherein the polycationic substance provides for uptake of the active compound across the mucosal surface and systemic distribution of the active compound and wherein (i) the substance is not a polyamino acid which chelates calcium or magnesium ions, (ii) the composition does not consist of microcapsules coated with DEAE-dextran, (iii) if for administration to gut mucosa, the composition does not consist of the active compound and a solution of DEAE-dextran and (iv), if in the form of a tablet for retention in the mouth, the composition does not comprise chitosan.

2. The use according to Claim 1 wherein the composition comprises microspheres of the polycationic substance.

3. The use according to Claim 1 wherein the composition comprises a solution or dispersion of the polycationic substance.

4. The use according to Claim 3 wherein the concentration of the polycationic substance is 0.01 to 50% w/v.

5. The use according to any other of the preceding claims wherein the pharmacologically active compound is insulin.

6. The use according to any one of the preceding claims wherein the polycationic substance is DEAE-dextran or chitosan or a salt or derivative thereof.

7. A complex of a pharmacologically active compound and chitosan or a salt or derivative thereof.

8. A complex according to Claim 7 for therapeutic use.

9. The use of a complex of a pharmacologically active compound and a polymeric substance having a plurality of cationic groups ("a polycationic substance") in the manufacture of a medicament for delivery to a mucosal surface wherein the polycationic substance provides for uptake of the active compound across the mucosal surface and systemic distribution of the active compound.

## Patentansprüche

1. Verwendung einer polymeren Substanz mit einer Mehrzahl von kationischen Gruppen und einem Molekulargewicht von 10000 oder mehr (im folgenden als "polykationische Substanz" bezeichnet) bei der Herstellung einer weiterhin eine pharmakologisch aktive Verbindung enthaltenden Zubereitung zur Verabreichung an eine Schleimhaut, wobei die polykationische Substanz für eine Aufnahme der aktiven Verbindung durch die Schleimhautoberfläche und eine systemische Verteilung der aktiven Verbindung sorgt und wobei gilt: (i) Die Substanz besteht nicht aus einer Calcium- oder Magnesiumionen chelatisierenden Polyaminosäure; (ii) die Zubereitung besteht nicht aus mit DEAE-Dextran beschichteten Mikrokapseln; (iii) wenn für eine Verabreichung an die Darmschleimhaut vorgesehen, besteht die Zubereitung nicht aus der aktiven Verbindung und einer Lösung von DEAE-Dextran und (iv) wenn in Form einer Tablette zur Rückhaltung im Mund vorliegend, enthält die Zubereitung kein Chitosan.

2. Verwendung nach Anspruch 1, wobei die Zubereitung Mikrokügelchen der polykationischen Substanz umfaßt.

3. Verwendung nach Anspruch 1, wobei die Zubereitung eine Lösung oder Dispersion der polykationischen Substanz umfaßt.

4. Verwendung nach Anspruch 3, wobei die Konzentration der polykationischen Substanz 0,01 bis 50% g/v beträgt.

5. Verwendung nach einem der vorhergehenden Ansprüche, wobei die pharmakologisch aktive Verbindung aus Insulin besteht.

6. Verwendung nach einem der vorhergehenden Ansprüche, wobei die polykationische Substanz aus DEAE-Dextran oder Chitosan oder einem Salz oder Derivat hiervon besteht.

7. Komplex aus einer pharmakologisch aktiven Verbindung und Chitosan oder einem Salz oder Derivat hiervon.

8. Komplex nach Anspruch 7 zum therapeutischen Gebrauch,

9. Verwendung eines Komplexes einer pharmakologisch aktiven Verbindung und einer polymeren Substanz mit einer Mehrzahl von kationischen Gruppen ("einer polykationischen Substanz") bei der Herstellung eines Arzneimittels zur Abgabe an eine Schleimhautoberfläche, wobei die polykationische Substanz für eine Aufnahme der aktiven Verbindung durch die Schleimhautoberfläche und eine systemische Verteilung der aktiven Verbindung sorgt.

## Revendications

1. Utilisation d'une substance polymère ayant plusieurs groupes cationiques et ayant un poids moléculaire de 10 000 ou plus (ci-après "substance polycationique") dans la fabrication d'une composition à administrer à une muqueuse, cette composition comprenant en outre un composé pharmacologiquement actif, dans laquelle la substance polycationique permet l'absorption du composé actif au travers de la surface des muqueuses et la distribution systémique du composé actif, et dans laquelle (i) la substance n'est pas un polyaminoacide qui se combine par chélation avec des ions calcium ou magnésium, (ii) la composition n'est pas constituée par des microcapsules revêtues avec du DEAE-dextrane, (iii) si elle est administrée aux muqueuses de l'intestin, la composition n'est pas constituée du composé actif et d'une solution de DEAE-dextrane et (iv), si elle est sous la forme d'un comprimé à conserver dans la bouche, la composition ne comprend pas de chitosane.

2. Utilisation selon la revendication 1, dans laquelle la composition comprend des microsphères de la substance polycationique.

3. Utilisation selon la revendication 1, dans laquelle la composition comprend une solution ou dispersion de la substance polycationique.

4. Utilisation selon la revendication 3, dans laquelle la concentration de la substance polycationique est de 0,01 à 50 % p/v.

5. Utilisation selon l'une quelconque des revendications précédentes, dans laquelle le composé pharmacologiquement actif est l'insuline.

6. Utilisation selon l'une quelconque des revendications précédentes, dans laquelle la substance polycationique est le DEAE-dextrane ou le chitosane ou un sel ou un dérivé de ceux-ci.

7. Complexe d'un composé pharmacologiquement actif et de chitosane ou un sel ou dérivé de celui-ci.

8. Complexe selon la revendication 7 à usage thérapeutique.

9. Utilisation d'un complexe d'un composé pharmacologiquement actif et d'une substance polymère ayant plusieurs groupes cationiques ("substance polycationique") dans la fabrication d'un médicament à délivrer à une surface de muqueuse, dans laquelle la substance polycationique permet l'absorption du composé actif au travers de la surface de muqueuse et la distribution systémique du composé actif.
